# FASCICULE DE BREVET EUROPEEN

(11) **EP 0 500 455 B1**
(45) Date de publication et mention de la délivrance du brevet: **12.06.1996**
(21) Numéro de dépôt: 92400430.2
(22) Date de dépôt: 18.02.1992
(51) Int. Cl.: A61K 7/30

(54) **Liquide de nettoyage pour prothèses dentaires**
Flüssiges Reinigungsmittel für Zahnprothesen
Liquid cleaning product for dentures

(30) Priorité: 18.02.1991 ES 9100422
(43) Date de publication de la demande: 26.08.1992
(73) Titulaire: Bueno Flamarique, Luis Miguel, E-31011 Pamplona (ES)
(72) Inventeur: Bueno Flamarique, Luis Miguel, E-31011 Pamplona (ES)
(74) Mandataire: Le Roux, Martine

(56) Documents cités:
- DE-A- 1 617 744
- ES-A- 8 602 687
- FR-A- 1 428 137
- FR-A- 2 356 600
- S.T.N., SERVEUR DE BASES DE DONNEES, Karlsruhe, DE, FICHIER CHEMICAL ABSTRACTS, vol. 111, no. 10, résumé no. 83918q, Columbus, Ohio, US; & ES-A- 86 02 687 (ESSENTIAL OILS AND COSMETICS) 20-10-1986
- S.T.N., SERVEUR DE BASES DE DONNEES, Karlsruhe, DE, FICHIER CHEMICAL ABSTRACTS, vol. 114, no. 2, résumé no. 12010p, Columbus, Ohio, US; & CN-A-1 041 101 (ESSENTIAL OILS AND COSMETICS) 11-04-1990

## Description

La présente invention concerne un liquide de nettoyage pour prothèses dentaires amovibles. Ce liquide est du type de celui décrit dans la demande ES-A-8602687.

Sur les prothèses dentaires, il apparaît au cours du temps certains dépôts calcaires couramment dénommés "tartre", dont l'origine est due à la chaux que contient la salive et dont le contact avec des résidus de nourriture, de vin, de tabac..., engendre des dépôts calcaires qui, sous forme de plaques, adhèrent aux dents et, par conséquent, à la denture postiche dénommée prothèse dentaire amovible.

A ce jour, pour nettoyer lesdites prothèses, on connaît l'emploi de divers produits qui, normalement, sont mélangés avec de l'eau, pour l'obtention d'un liquide dans lequel on introduit lesdites prothèses On les laisse pendant un certain temps.

Ces solutions aqueuses, normalement, ne font qu'oxygéner l'eau avec une intensité et une efficacité plus ou moins grandes, ce qui facilite le détachement des résidus alimentaires déposés sur les prothèses, mais n'agit pas contre le tartre et donc la quantité de celui-ci augmente au cours du temps bien que l'on utilise de tels moyens de nettoyage. Ceci oblige finalement à recourir à un processus laborieux de nettoyage réalisé normalement par un professionnel comme le prothésiste dentaire.

L'objet de la présente invention est un liquide de nettoyage qui assure l'élimination non seulement des impuretés adhérant à une prothèse dentaire, mais aussi du tartre et en totalité ; au moyen d'un procédé qui consiste seulement à introduire la prothèse dans ledit liquide de nettoyage pendant une durée ne dépassant pas 8 h et à rincer ensuite abondamment à l'eau.

Selon l'invention, le liquide de nettoyage comprend avantageusement quatre composants de base qui sont : l'eau distillée ; l'acide chlorhydrique, le peroxyde d'hydrogène et au moins un conservateur.

Avec ces composants et en introduisant la prothèse dentaire dans le liquide de nettoyage, de manière que celui-ci la recouvre totalement et que le récipient dans lequel est réalisé le procédé soit de préférence fermé pour améliorer l'efficacité du liquide de nettoyage, il se développe un procédé au cours duquel l'acide chlorhydrique ramollit tout d'abord le tartre en transformant sa structure de plaques dures en une composition ramollie et poreuse. Dans cette situation, on atteint les conditions idoines pour que le peroxyde d'hydrogène puisse agir, en pénétrant dans les porosités ainsi produites dans les dépôts de tartre, ce qui produit le détachement de ce dernier, de lui-même ou bien au moyen du simple brossage et du lavage que l'on doit réaliser à la fin du procédé.

Le liquide de nettoyage de la présente invention est tel que revendiqué dans le jeu de revendications annexé.

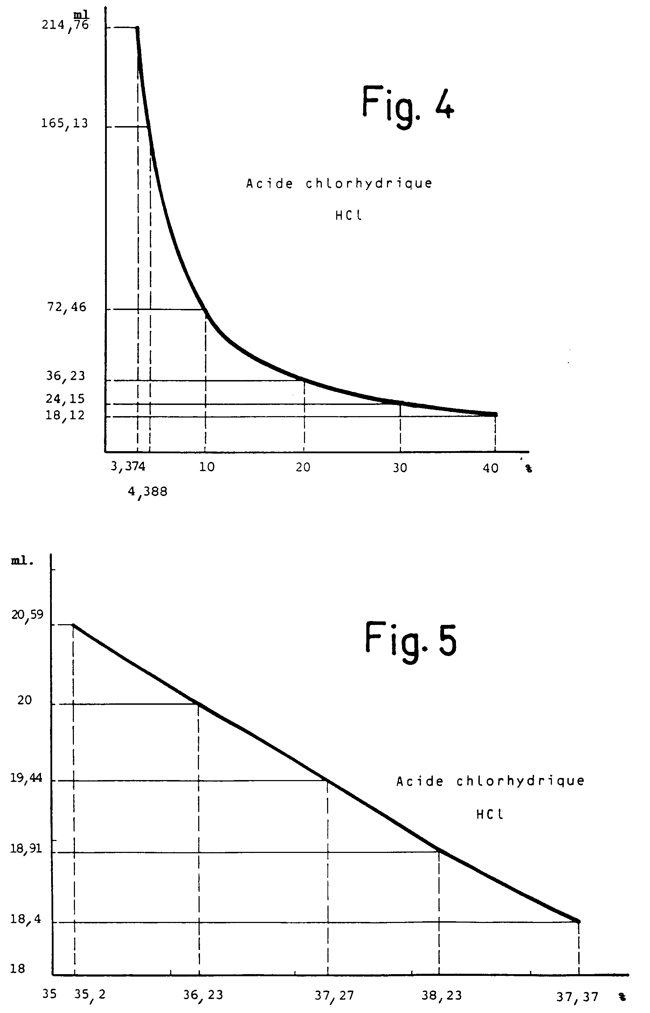

En ce qui concerne l'acide chlorhydrique, le volume qui intervient est fonction de sa teneur (exprimée en pourcentage en poids). La relation volume/teneur au sein des solutions de l'invention est définie sur la Fig. 4. Le pH des solutions de l'invention est compris entre 0,5 et 1,8. Avantageusement, il est voisin de 1,5.

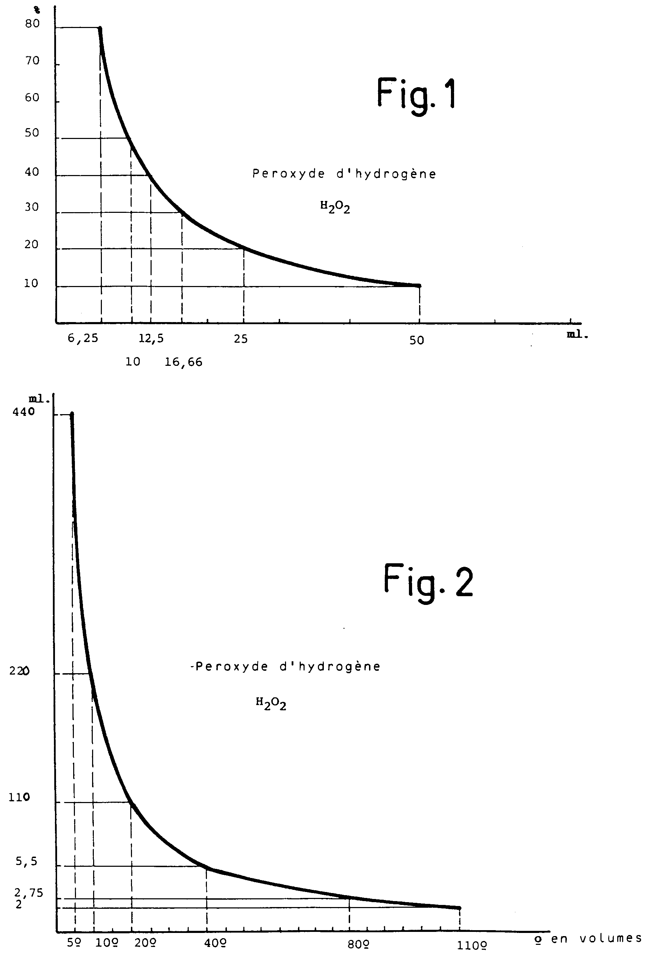

En ce qui concerne le peroxyde d'hydrogène, le volume qui intervient est fonction de sa teneur (exprimée en pourcentage en poids) ou de son volume en degré. Les relations volume/teneur, volume/volume en degré sont avantageusement celles définies sur les Fig. 1 à 3. Les solutions de l'invention renferment dans 1 000 ml, de 1 à 15 ml d'H₂O₂ à 50 % en poids ou une quantité équivalente d'H₂O₂. Avantageusement, elles renferment dans 1 000 ml environ 10 ml d'H₂O₂ à 50 % en poids ou une quantité équivalente.

L'invention sera mieux comprise à la lecture de la description qui va suivre d'un mode de mise en oeuvre industriel préféré, en référence aux dessins annexés sur lesquels :
la figure 1 est un graphique du peroxyde d'hydrogène pour une solution selon l'invention de 1 000 ml, représentant en abscisses le volume en ml et en ordonnées, la teneur en % en poids.
La figure 2 est un graphique du peroxyde d'hydrogène, également pour une solution selon l'invention de 1 000 ml, mais représentant en abscisses les volumes en degrés et en ordonnées, le volume en ml.
La figure 3 est un graphique qui représente à la fois les deux graphiques précédents.
La figure 4 représente un graphique de l'acide chlorhydrique, également pour une solution selon l'invention de 1 000 ml, représentant en ordonnées le volume en ml et en abscisses, la teneur en % en poids.
La figure 5 correspond à une vue en détail de la partie du graphique de la figure 4 comprise entre 35 et 40 % en poids.

L'objet de la présente invention est un liquide de nettoyage pour prothèse dentaires amovibles.

Dans ce liquide, les composants qui le forment et les pourcentages de ces composants sont fondamentaux.

Les figures 1 à 5 ci-annexées correspondent à des graphiques qui montrent les différentes valeurs et proportions de ces composants pour une solution de 1 000 ml.

Ainsi, la figure 1 correspond à un graphique du peroxyde d'hydrogène pour une solution de 1 000 ml représentant en ordonnées la teneur en % en poids et en abscisses, le volume en ml. Quelques-unes des valeurs qui ont permis d'établir ce graphique sont les suivantes :

| | |
|---|---|
| 80 % en poids de H₂O₂ | 6,25 ml |
| 50 % en poids de H₂O₂ | 10 ml |
| 40 % en poids de H₂O₂ | 12,5 ml |
| 30 % en poids de H₂O₂ | 16,66 ml |
| 20 % en poids de H₂O₂ | 25 ml |
| 10 % en poids de H₂O₂ | 50 ml. |

La figure 2 correspond à un graphique du peroxyde d'hydrogène représentant les volumes en ml en ordonnées et les volumes en degrés de peroxyde d'hydrogène en abscisses. Las valeurs indiquées sont les suivantes :

| | |
|---|---|
| 5° en volume | 440 ml |
| 10° en volume | 220 ml |
| 20° en volume | 110 ml |
| 40° en volume | 55 ml |
| 80° en volume | 27,5 ml |
| 110° en volume | 20 ml |
| 220° en volume | 10 ml |

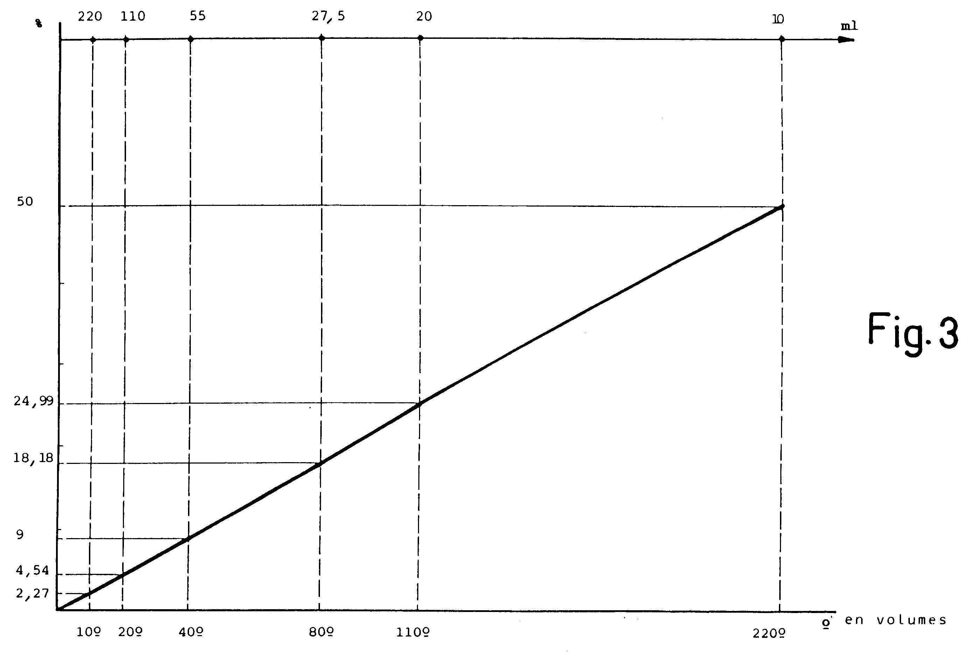

La figure 3 représente un graphique du peroxyde d'hydrogène représentant à la fois les volumes en degrés, la teneur en % en poids et les volumes en ml ; il en résulte un graphique qui nous indique que, par exemple avec un peroxyde d'hydrogène à 100° en volume et 24,99 % en poids, il faut 20 ml pour une solution de 1 000 ml selon l'invention, tandis qu'avec un peroxyde d'hydrogène à 220° en volume et 50 % en poids, il faut 10 ml pour la même solution selon l'invention de 1 000 ml.

La figure 4 correspond à un graphique de l'acide chlorhydrique, également pour une solution selon l'invention de 1 000 ml. Les ordonnées représentent le volume de HCl en ml et les abscisses, sa teneur en % en poids. La figure 5 représente agrandie la partie du graphique précédent comprise entre 35 et 40 % en poids.

Les valeurs représentées sont les suivantes :

| | |
|---|---|
| 40 % en poids | 18,12 ml |
| 39,37 % en poids | 18,40 ml |
| 38,32 % en poids | 18,91 ml |
| 37,27 % en poids | 19,44 ml |
| 36,23 % en poids | 20 ml |
| 35,20 % en poids | 20,59 ml |
| 4,388 % en poids | 165,13 ml |
| 3,374 % en poids | 214,76 ml |
| 2,364 % en poids | 306,51 ml |
| 1,360 % en poids | 532,79 ml. |

Dans la pratique, on a démontré que pour obtenir les meilleurs résultats, toujours pour une solution de 1 000 ml, l'acide chlorhydrique doit aller d'un minimum de 20 ml à un maximum d'environ 50 ml.

La peroxyde d'hydrogène doit aller d'un minimum de 10 ml à un maximum de 25 ml.

D'autre part, le rapport préféré entre l'acide chlorhydrique et le peroxyde d'hydrogène est de 2 à 1, respectivement.

Dans ces conditions, une solution de 1 000 ml de liquide de nettoyage donnant des résultats efficaces peut être préparée selon les pourcentages et caractéristiques suivants :

| | |
|---|---|
| eau distillée | 920 ml |
| acide chlorhydrique à 18 % en poids | 40 ml |
| peroxyde d'hydrogène à 100° en volume | 20 ml |
| conservateurs | 20 ml. |

Les conservateurs sont avantageusement des esters de l'acide parahydroxybenzoïque ou leurs sels, tel que le sel de sodium du p-hydroxybenzoate de méthyl ou bien le sorbate de potassium (ou leurs mélanges).

Conformément à toutes ces valeurs, on indique ci-après quelques exemples possibles de préparation du liquide de nettoyage, à titre d'exemples non limitatifs de réalisation pratique de l'invention.

### Exemple 1

On prépare 1 000 ml du liquide de nettoyage, dont les composants présentent les caractéristiques suivantes :

| | |
|---|---|
| eau (H₂O) | eau distillée |
| peroxyde d'hydrogène (H₂O₂) | à 100° en volume |
| acide chlorhydrique (HCl) | à 36,23 % en poids |
| conservateurs | sels des esters de l'acide parahydroxybenzoïque ou bien sorbate de potassium |

Dans ce cas, sur ledit total de 1 000 ml, le volume des composants est :

| | |
|---|---|
| H₂O | 940 ml |
| HCl | 20 ml |
| H₂O₂ | 20 ml |
| conservateurs | 20 ml. |

### Exemple 2

On prépare 1 000 ml du liquide de nettoyage dont les composants présentent les caractéristiques suivantes :

| | |
|---|---|
| eau (H₂O) | eau distillée |
| peroxyde d'hydrogène (H₂O₂) | à 40° en volume |
| acide chlorhydrique (HCl) | à 4,388 % en poids |
| conservateurs | sels d'esters de l'acide parahydroxybenzoïque ou bien sorbate de potassium |

Le volume des composants est :

| | |
|---|---|
| H₂O | 759,87 ml |
| H₂O₂ | 55 ml |
| HCl | 165,13 ml |
| conservateurs | 20 ml. |

### Exemple 3

On prépare 1 000 ml du liquide de nettoyage dont les composants présentent les caractéristiques suivantes :

| | |
|---|---|
| eau (H₂O) | eau distillée |
| peroxyde d'hydrogène (H₂O₂) | à 5° en volume |
| acide chlorhydrique (HCl) | à 40 % en poids |
| conservateurs | sels des esters de l'acide parahydroxybenzoïque ou bien sorbate de potassium |

Le volume des composants est :

| | |
|---|---|
| H₂O | 521,88 ml |
| H₂O₂ | 440 ml |
| HCl | 18,12 ml |
| conservateurs | 20 ml. |

### Exemple 4

On prépare 1 000 l d'un liquide de nettoyage selon l'invention en mélangeant :
10 l d'H₂O₂ à 220° en volume (ou 50 % en poids)
20 l d'HCl à 36,23 % en poids
125 g de méthyl p-oxybenzoate de sodium
970 l d'eau distillée

Les 1 000 l obtenus ont un pH de 1,5 et contiennent 0,72 mol/l de conservateur.

## Revendications

1. Liquide de nettoyage pour prothèses dentaires amovibles, à base de l'eau distillée (H₂O), d'acide chlorhydrique (HCl) et de peroxyde d'hydrogène (H₂O₂), caractérisé en ce que ladite solution :
- présente un pH compris entre 0,5 et 1,8 ;
- a une teneur en H₂O₂ équivalente à l'une de celles d'une solution aqueuse de 1000 ml contenant de 1 à 15 ml d'H₂O₂ à 50 % en poids.

2. Liquide de nettoyage selon la revendication 1, caractérisé en ce que ladite solution a un pH voisin de 1,5.

3. Liquide de nettoyage selon l'une des revendications 1 ou 2, caractérisé en ce que ladite solution renferme dans 1000 ml environ 10 ml d'H₂O₂ à 50 % en poids ou une quantité équivalente.

4. Liquide de nettoyage selon l'une quelconque des revendications 1 à 3, caractérisé en ce que ladite solution contient une quantité efficace d'au moins un conservateur.

5. Liquide de nettoyage selon la revendication 4, caractérisé en ce que ledit conservateur est choisi parmi les esters de l'acide parahydroxybenzoïque et leurs sels, le sorbate de potassium et leurs mélanges.

6. Liquide de nettoyage selon l'une des revendications 4 ou 5, caractérisé en ce que ladite solution renferme une quantité efficace du sel de sodium du p-hydroxybenzoate de méthyl.

7. Liquide de nettoyage selon l'une quelconque des revendications 1 à 6, caractérisé en ce que l'eau entrant dans sa composition est de l'eau distillée.

8. Liquide de nettoyage selon l'une quelconque des revendications 1 à 7, caractérisé en ce qu'il renferme, pour 1000 l :
10 l de peroxyde d'hydrogène à 50 % en poids (ou 220° en volume)
20 l d'acide chlorhydrique à 36,23 % en poids
125 g de méthyl p-hydroxybenzoate de sodium
970 l d'eau distillée.

## Claims

1. Cleaning liquid for removable dentures, based on distilled water (H₂O), hydrochloric acid (HCl) and hydrogen peroxide (H₂O₂), characterized in that the said solution:
- has a pH of between 0.5 and 1.8;
- has an H₂O₂ content equivalent to one of those of an aqueous solution of 1000 ml containing from 1 to 15 ml of 50 % H₂O₂ by weight.

2. Cleaning liquid according to Claim 1, characterized in that the said solution has a pH in the region of 1.5.

3. Cleaning solution according to either of Claims 1 and 2, characterized in that the said solution contains, in 1000 ml, about 10 ml of 50 % H₂O₂ by weight or an equivalent amount.

4. Cleaning liquid according to any one of Claims 1 to 3, characterized in that the said solution contains an effective amount of at least one preserving agent.

5. Cleaning liquid according to Claim 4, characterized in that the said preserving agent is chosen from para-hydroxybenzoic acid esters and salts thereof, potassium sorbate and mixtures thereof.

6. Cleaning liquid according to either of Claims 4 and 5, characterized in that the said solution contains an effective amount of the sodium salt of methyl p-hydroxybenzoate.

7. Cleaning liquid according to any one of Claims 1 to 6, characterized in that the water forming part of the composition thereof is distilled water.

8. Cleaning liquid according to any one of Claims 1 to 7, characterized in that it includes, per 1000 l:
10 l of 50 % hydrogen peroxide by weight (or 220 volumes)
20 l of 36.23 % hydrochloric acid by weight
125 g of sodium methyl p-hydroxybenzoate
970 l of distilled water.

## Patentansprüche

1. Reinigungsflüssigkeit für herausnehmbare Zahnprothesen auf der Basis von destilliertem Wasser (H₂O), Salzsäure (HCl) und Wasserstoffperoxid (H₂O₂),
dadurch gekennzeichnet, daß
die Lösung:
- einen pH-Wert im Bereich von 0,5 bis 1,8 und
- einen Gehalt an H₂O₂ aufweist, der dem Gehalt einer wäßrigen Lösung von 1000 ml, die 1 bis 15 ml H₂O₂-Lösung von 50 Gew.-% enthält, entspricht.

2. Reinigungsflüssigkeit nach Anspruch 1, dadurch gekennzeichnet, daß die Lösung einen pH-Wert von etwa 1,5 aufweist.

3. Reinigungsflüssigkeit nach Anspruch 1 oder 2, dadurch gekennzeichnet, daß die Lösung in 1000 ml etwa 10 ml H₂O₂-Lösung von 50 Gew.-% oder eine äquivalente Menge davon enthält.

4. Reinigungsflüssigkeit nach einem der Ansprüche 1 bis 3, dadurch gekennzeichnet, daß die Lösung eine wirksame Menge mindestens eines Konservierungsmittels enthält.

5. Reinigungsflüssigkeit nach Anspruch 4, dadurch gekennzeichnet, daß das Konservierungsmittel unter den p-Hydroxybenzoesäureestern und ihren Salzen, Kaliumsorbat und deren Gemischen ausgewählt ist.

6. Reinigungsflüssigkeit nach Anspruch 4 oder 5, dadurch gekennzeichnet, daß die Lösung eine wirksame Menge des Natriumsalzes des p-Hydroxybenzoesäuremethylesters enthält.

7. Reinigungsflüssigkeit nach einem der Ansprüche 1 bis 6, dadurch gekennzeichnet, daß das Wasser in der Zusammensetzung destilliertes Wasser ist.

8. Reinigungsflüssigkeit nach einem der Ansprüche 1 bis 7, dadurch gekennzeichnet, daß sie auf 1000 l enthält:
10 l Wasserstoffperoxid von 50 Gew.-% (oder 220 Vol.-°),
20 l Salzsäure von 36,23 Gew.-%,
125 g Natrium-p-hydroxybenzoesäuremethylester,
970 l destilliertes Wasser.
